# EUROPEAN PATENT SPECIFICATION

(11) **EP 3 749 234 B1**
(45) Date of publication and mention of the grant of the patent: **29.11.2023**
(21) Application number: 19715978.3
(22) Date of filing: 11.02.2019
(51) Int. Cl.: A61B 17/34

(54) **MEDICAL DEVICE FOR THE DELIVERY OF THERAPEUTIC FORMULATIONS**
MEDIZINPRODUKT ZUR ABGABE THERAPEUTISCHER FORMULIERUNGEN
DISPOSITIF MÉDICAL POUR L'ADMINISTRATION DE FORMULATIONS THÉRAPEUTIQUES

(30) Priority: 09.02.2018 PT 2018110563
(43) Date of publication of application: 16.12.2020
(73) Proprietor: Stemmatters, Biotecnologia e Medicina Regenerativa, S.A., 4805-017 Barco GMR (PT)
(72) Inventor: VILELA GOMES, Carlos Alberto, 4705-303 Braga (PT); ROMERO AMANDI DE SOUSA, Rui Pedro, 4450-088 Matosinhos (PT); CORREIA, Cristina, 4730-500 São Mamede Escariz (PT); GONÇALVES DOS REIS, Rui Luís, 4250-242 Porto (PT); COELHO DO SAMEIRO ESPREGUEIRA MENDES, João Duarte, 4150-326 Porto (PT)
(74) Representative: Patentree
(86) International application number: PCT/IB2019/051083
(87) International publication number: WO 2019/155434

(56) References cited:
- EP-A1- 1 656 960
- WO-A1-2013/084943
- US-A1- 2009 060 974

## Description

### TECHNICAL FIELD

This disclosure relates to a medical device for delivering therapeutic or diagnostic formulations to articular joints and, more preferably, for delivering therapeutic formulations to articular cartilage defect sites.

### BACKGROUND

Articular cartilage defects are present in a majority of patients undergoing arthroscopic procedures (Widuchowski et al. 2007). The high prevalence of articular cartilage defects is related to the limited endogenous repair capacity of cartilage. The body repairs damaged cartilage by filing the lesion site with fibrocartilage. The fibrocartilage scar tissue formed, as compared to normal hyaline articular cartilage, has inferior mechanical and biochemical properties. This fibrocartilage scar tissue contains significant amounts of collagen type I, does not function well as a cushion between joints and is more susceptible to degeneration. The loading and breakdown of this scar tissue can eventually lead to premature osteoarthritis. The aim of current surgical treatment procedures is to promote a more effective cartilaginous response by using marrow stimulating techniques, such as microfracture (Mithoefer et al. 2005). These procedures, generally considered first-line treatments for focal defects, are cost-effective and clinically useful because patients often experience reduced pain and improved joint function. However, in most cases, the relief patients experience are only temporary. Hence, the long term therapeutic effect of current surgical treatment procedures is questionable (Benthien & Behrens 2011).

The aim of current therapeutic procedures based on cell or tissue transplantation is to achieve regeneration of hyaline-like cartilage tissue. Autologous Chondrocyte Implantation (ACI) procedure is the most widely used therapeutic procedure (Brittberg et al. 1994). First generation ACI involves the use of a periosteal flap or a collagen sheet which is fixed to the surrounding cartilage to create a reservoir for injecting a suspension of ex vivo expanded autologous chondrocytes (Peterson et al. 2000; Brittberg et al. 1994). Currently, ACI is the only clinically available cell-based therapy for cartilage repair. ACI procedure may be inadequate in certain scenarios because of anatomic factors and/or difficulty in fixing the periosteal flap or collagen sheets to retain the chondrocyte suspension in the cartilage defect site. ACI procedures are prone to surgical complications; 25% to 36% of patients treated by ACI procedures require further surgeries (Harris et al. 2011). In order to overcome the disadvantages of first generation ACI, attempts have been made to adopt cartilage tissue engineering strategies based on the use of scaffolds or matrices. Cartilage grafts are reconstructed by using a scaffold on which autologous chondrocytes can proliferate threedimensionally (MACI - Matrix-induced autologous chondrocyte implantation) (Basad et al. 2010). The limitations of current methods have led to the use of alternative matrices in combination with therapeutic cells for cartilage repair. An attractive approach is the use of cell encapsulation platforms by which cells are delivered and retained at the focal cartilage defect site. In this regard, hydrogels present appealing properties for tissue engineering and regenerative medicine applications as they swell and retain large amounts of water, are tissue mimetic and can be delivered using minimally invasive procedures (i.e., injection) (Lum et al. 2003). In addition, hydrogels provide a highly hydrated microenvironment (resembling extracellular matrix - ECM) and can be crosslinked in situ allowing cell encapsulation and preservation/induction of their differentiated phenotype.

In spite of developments at research level, limited improvements have been made at clinical level in relation to surgical techniques used to deliver these combination products into cartilage defects and ensure retention within the lesion site.

The procedures used to treat cartilage defects typically involve arthrotomy or arthroscopy procedures (Carr et al. 2014). Arthrotomy procedures involve longer surgical intervention duration and higher cost of the treatment. Arthroscopy procedures can reduce surgical cost and enable outpatient treatment. Arthroscopy procedures have been used in the treatment of cartilage defects by using MACI (Ibarra et al. 2014; Vascellari et al. 2014). Briefly, the MACI construct is inserted through a portal by means of a cannula or forceps and fixed using additional fixation means such as a bioabsorbable suture (Ibarra et al. 2014) or fibrin glue (Vascellari et al. 2014). As the use of an expansion liquid can compromise integrity of MACI construct and impede its fixation, the inflow of expansion liquid is stopped. This cause collapse of the joint and impedes further internal visualization. To avoid this, during the implantation procedure the expansion liquid is temporarily stopped, the articular cavity collapses to its normal position, and an expansion gas is used to expand the articular cavity again for implantation of the construct.

Controlled spatiotemporal delivery of therapeutic formulations to the defect site is an important requirement in arthroscopy as it increases therapeutic efficacy and efficiency by ensuring confined localized delivery to the defect site and avoids inadvertent loss of valuable therapeutic formulation to the surrounding articular cavity. The present problem to be solved thus involves the controlled spatiotemporal delivery of therapeutic formulations by a minimally invasive procedure such as arthroscopy. The intention is to allow uninterrupted treatment of the cartilage defect site by ensuring continuous expansion of the articular cavity. This problem is of special relevance taking into account that the arthroscopic procedure requires injection of an expansion liquid or gas into the cavity space. The expansion fluid or gas can negatively interfere with the therapeutic formulation being applied. As such, the technical solution should limit the delivery of a therapeutic formulation exclusively to the defect site, impede its leakage to the surrounding articular cavity and avoid the invasion of the cartilage defect site by the fluid used for articular cavity expansion. In addition, the technical solution should be compatible with the expansion medium, which may be either a liquid or gas, so as to avoid the need for double step articular cavity expansion procedure. The difficulty of this challenge is increased for low viscosity therapeutic formulations as these formulations may easily flow out of the defect site and spread throughout the surrounding articular cavity during the implantation procedure.

Other methods and devices have been developed with the intention of improving surgical efficacy of treatment methods by means of arthroscopic procedures.

For example, document WO2005079881 describes an arthroscopic method for scaffold-free cell implantation in mammals, whereby the filling of a cartilage defect is made by a catheter injection of a cell suspension using a portal. In order to fix the cells into a defect, the cell suspension is allowed to settle under the influence of gravity. The adhesion of the cell suspension is ensured by adjusting viscosity and concentration of divalent cations. However, this document does not provide a technical solution for the abovementioned problem, especially taking into consideration that the articular cavity becomes inflated by the expansion fluid that can easily remove the cell suspension from the defect site.

Document US20050137600 describes surgical instruments for preparation of an articular cartilage site for arthroscopic implantation of an articular cartilage repair device into subchondral bone.

Document US2007077236 describes a method wherein a cell suspension is applied through a portal into the articular joint by injection, this is followed by the sedimentation of cultured cells and adhesion of cells to the subchondral bone and/or cartilage. In this document, the media with cells is applied simultaneously with a support material during an arthroscopic procedure. The mixing of these components occurs during the flow displacement, and upon application, the support material coagulates. The proposed method claims the use of a sealant material over the coagulated cell/support. However, this method does not limit the application of cell suspension and support material to the defect site, neither does it impedes the migration of the cell suspension, the support material or the sealant material to the surrounding articular cavity.

Document EP1656960 discloses the arthroscopic delivery of cells seeded in a foldable support matrix by means of an introducer featuring a telescopic working channel terminated by a cap. The device to be implanted is delivered by folding it conveniently through a channel. However, the application of this device is not trivial as the unfolding and fixation of the implantable device in the cavity raises manipulation challenges that extends the duration of the surgical procedure and increase likelihood of procedure variability by different surgeons. Taking into consideration the demanding requirements imposed by material characteristics and the biological requirements of cells being co-delivered to the defect site, the injection and fixation of a hydrogel-based formulation is not technically convenient using any of the devices.

Document WO2013155359 discloses a two-needle device featuring a cannula/needle and a longer inner needle which can be used as a delivery system for stimulation of the bone chondral interphase. Although the system can be used to deliver certain therapeutic agents into the articular cavity, it would not be possible to use such system to address the challenge pertaining to delivery of a therapeutic formulation comprising for example, biomaterial matrices or therapeutic formulations consisting of combinations involving these.

Document US2012283833 also describes a method for diagnosing and treating articular related bone conditions. This method applies to conditions in which the underlying bone is physiologically impaired, which does not necessarily apply to all types of chondral defects.

WO2013/084943 A1 and US2009/060974 A1 disclose a catheter assembly and an umbrella device, respectively.

These facts are disclosed in order to illustrate the technical problem addressed by the present disclosure.

### GENERAL DESCRIPTION

The invention is defined in claim 1. Preferred embodiments are specified in the dependent claims.

The present disclosure describes a device for delivery of therapeutic formulations to the articular joint and, more particularly to an articular cartilage defect in a human or animal subject.

The present disclosure further describes a device and method (not claimed) for endoscopic implantation of therapeutic formulations into a tissue or tissue defect of a human or mammalian animal.

The device is in particular intended to be used during an arthroscopy procedure to deliver a therapeutic formulation into a cartilage defect by an arthroscopic port. The device allows a continuous connection between the cartilage defect cavity and an aseptically controlled environment outside the body. According to an aspect of the disclosure, the device and all its components have a distal end and a proximal end. The distal end is intended to be used inside the articular cavity while the proximal end faces the surgeon and provides access to the interior of the device. In a preferred embodiment, the device exhibits enough flexibility to allow easy entering and manoeuvring within the articular cavity during the arthroscopy procedure.

As an aspect (not claimed) of the disclosure, an injectable hydrogel should preferably present a sol-gel transition mechanism suitable for clinical purposes, i.e., it should be liquid to facilitate homogeneous cell distribution and injection, while being capable of rapidly solidifying after implantation and adhering to a cartilage defect.

The disclosed minimally invasive procedure (not claimed), and respective device, address cartilage defects benefitting the treatment of patients, providing an operationally simplified procedure that enables reduction in time and co-morbidity, which may ultimately support outpatient treatment regime and lower cost of treatment.

### BRIEF DESCRIPTION OF THE DRAWINGS

The following figures provide preferred embodiments for illustrating the disclosure and should not be seen as limiting the scope of this disclosure:
**Figure 1****:** Schematic representation of an embodiment of the distal section of the device.
**Figure 2****:** Exploded view of an embodiment of the device featuring its main components.
**Figure 3****:** Schematic representation of an embodiment of the external sleeve of the device.
**Figure 4****:** Schematic representation of an embodiment of the cup.
**Figure 5****:** Schematic representation of an embodiment of the delivery arm featuring four internal channels.
**Figure 6****:** Schematic representation of an embodiment of the cross-section of the delivery arm.
**Figure 7****:** Schematic representation of an embodiment of the gradual expansion of the cup.
**Figure 8****:** Schematic representation of an embodiment of the proximal section of the device.
**Figure 9****:** Schematic representation of an embodiment of the device featuring connection tubes and connection ports.
**Figure 10****:** Picture of a three-dimensional model of an embodiment of an articular knee joint and use of the device to treat two of its cartilage defect sites.
**Figure 11****:** Schematic representation of an embodiment of articular cartilage defect.
**Figure 12****:** Schematic representation of a cross-section of an articular cartilage defect, surrounding cartilage and, subchondral bone.
**Figure 13****:** Schematic representation of the perspective view of an embodiment of the cup covering an articular cartilage defect.
**Figure 14****:** Schematic representation of the cross-section of an embodiment of the cup covering an articular cartilage defect and the cartilage defect volume.
**Figure 15****:** Schematic representation of the cross-section of an embodiment of the cup covering an articular cartilage defect after filling of cartilage defect volume with therapeutic formulation.
**Figure 16****:** Schematic representation of the side view of an embodiment of the cup in two different heights.
**Figure 17****:** Schematic representation of an embodiment of the cup: side view and cross-section view with concave and convex interior surface.
**Figure 18****:** Schematic representation of the cross-section view of an embodiment of the cup and the angle between the axis and the edge of the cup.
**Figure 19****:** Schematic representation of the arthroscopic implantation of therapeutic formulations into an articular cartilage defect.
**Figure 20****:** Arthroscopic image of the arthroscopic implantation of therapeutic formulations into an articular cartilage defect in a cadaveric model: device being juxtaposed to a cartilage defect.
**Figure 21****:** Arthroscopic image of the arthroscopic implantation of therapeutic formulations into an articular cartilage defect in a cadaveric model: expansion of the cup.
**Figure 22****:** Arthroscopic image of the arthroscopic implantation of therapeutic formulations into an articular cartilage defect in a cadaveric model: covering of the cartilage defect site by the expanded cup and subsequently delivering a therapeutic formulation to the cartilage defect site.
**Figure 23****:** Arthroscopic image of the arthroscopic implantation of therapeutic formulations into an articular cartilage defect in a cadaveric model: removal of the cup.
**Figure 24****:** Arthroscopic image of the arthroscopic implantation of therapeutic formulations into an articular cartilage defect in a cadaveric model: therapeutic formulation delivered to the site.

### DETAILED DESCRIPTION

This disclosure relates to a device and method of use thereof (not claimed), for delivering therapeutic or diagnostic formulations to an articular joint and, more preferably, for delivering therapeutic formulations to articular cartilage defect sites.

Therapeutic formulations are defined as single components or combinations of different components in appropriate relationships, structures, and physical states used for diagnostic, prognostic or treatment of a tissue or organ.

A fluid vehicle is defined as the liquid phase used for displacement of the constituents of a therapeutic formulation.

A matrix is defined as the extracellular material used for cell seeding and cultivation. Upon controlled physicochemical and mechanical performance and, appropriate biological interaction, the matrix favours and promotes intended tissue regeneration.

A hydrogel is defined as a network of polymer chains in which water is the dispersion medium that can be displaced in liquid or viscous (pseudo-solid) forms and injected into the orifices, internal cavities or internal lesions in the body.

Cells are defined as a structural, functional, and biological unit of a living animal, including humans and animals. Chondrocytes are defined as the cells normally populating healthy cartilaginous tissues. Stem cells, including adult, embryonic and induced pluripotent stem cells, are defined as unspecialized cells capable of renewing themselves through cell division, which, under certain physiologic or experimental conditions, can be induced to become tissue or organ-specific cells. Chondrocyte progenitor cells are defined as early descendants of stem cells committed to become chondrocyte cells and produce cartilage tissue. Stem cells, chondrocyte progenitor cells, and chondrocytes are defined as cartilage forming cells.

A therapeutic drug is defined as a drug directed to enhance, modify, or maintain a biological or physiological function for the treatment, cure, prevention or diagnosis of disease. A biomarker is defined as a measurable indicator of biological state or condition of biological tissues. In one embodiment, the biomarker is an indicator of the biological state or condition of cartilage and surrounding tissues, including but not limited to bone or perichondrium.

A biomolecule is defined as any molecule that is normally present in or expressed by living organisms, or genetically modified organisms, which include, but are not limited to large macromolecules such as proteins, carbohydrates, lipids, and nucleic acids.

A cartilage defect is defined as a cartilage injury in a joint, such as the knee, which includes partialthickness to full-thickness lesions of the cartilage tissue that may also involve, depending on the severity of the defect, exposure, damage or surface cavitation/fissuring of the subchondral bone. The severity of the cartilage defect can be scored according to different scales or classifications, such as, for example, ICRS Clinical Cartilage Injury Evaluation system.

An endoscope is defined as a device that can be used for visual examination of a tissue surface through a port, without the need for open surgery. A port is defined as a small orifice created in the skin and underlying tissues for introduction of the endoscope. An arthroscope is defined as a special type of endoscope used within an articular joint to observe bone or cartilage surfaces. The arthroscope may comprise fibre optics, light source and specific lenses elements that enable examination of the joint without open surgery.

The device comprises a semi-flexible external sleeve (1), an internal sleeve (2) and, a flexible delivery arm (3). The external sleeve, the internal sleeve and, the delivery arm are concentric tubes. The external sleeve is in the shape of a tube with a lumen. In an equally preferred embodiment, the external sleeve is made up of interconnected rigid rings hence rendering the sleeve flexible.

The internal sleeve (2), is in the shape of a tube with a lumen and it moves along its main axis within the external sleeve. The flexible delivery arm (3) and the internal sleeve are connected concentric tubes that move as a unit. At its distal end, the delivery arm features a conical flexible cup (4), which upon axial movement of the delivery arm towards its distal end, is exposed outside the external sleeve and allows for expansion of the cup to its normal shape.

At its distal end, the cup can be placed juxtaposed to the cartilage defect so as to cover the cartilage defect. The cup has an interior (5) and an exterior surface (6). The interior surface of the cup is predominantly in contact with the cartilage surface while the exterior surface is in contact with synovial and expansion fluids present during arthroscopy procedures. The cup is pliable; it adjusts its shape to ensure a good fit on the cartilage surface that surrounds the defect. In one embodiment, the internal surface of the cup is in direct contact with the cartilage thus creating a tight seal between the cartilage surface and the cup. This creates a confined volume (18) between the cup and the cartilage defect (15) and isolates the cartilage defect from the surrounding fluid contained in the articular cavity.

In one embodiment, the displacement of the therapeutic formulation in the internal channel is ensured by a positive pressure applied at the proximal end. In an alternative embodiment, the displacement of the therapeutic formulation is assured by a movable plunger (8) that moves coaxially inside the internal channel and impedes accumulation of the therapeutic formulation along its path.

In one embodiment, the cup at its distal end has a circular shape when expanded to its normal shape. In one embodiment, the cup at its distal end is eccentric as it displays a non-circular ellipsoid shape when expanded to its normal shape. In one embodiment, the edge (22) of the cup at its distal end is planar. In another embodiment, the edge of the cup at its distal end is non-planar.

At its proximal section, the device features connection tubes (9) that are connected to the internal channels. The connection tubes (9) terminate in connection ports (10) which are compatible with standardized connectors such as Luer systems (for example, see ISO 80369-7:2016 - Small-bore connectors for liquids and gases in healthcare applications -- Part 7: Connectors for intravascular or hypodermic applications) or other connectors with equivalent purpose. The connection tubes are multi-purpose.

In a preferred embodiment, the device features Luer connectors, including double connectors (11). In one embodiment, the connectors are suitable for being
used for connecting syringes (20) or other containers comprising therapeutic formulations. In another embodiment, the connection tubes are suitable for being
used for administration and/or removal of fluids. In one embodiment, connection tubes and connectors are suitable for being
used for transmission of light or other non-ionizing radiation. In one embodiment, connection tubes are suitable for being
used for visualization of articular cavity.

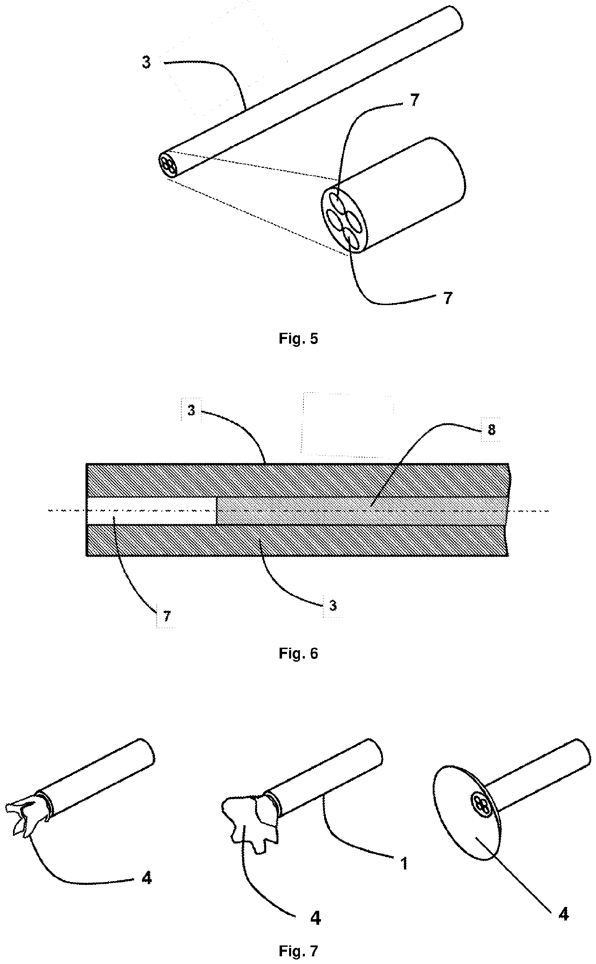

The delivery arm has more than one internal channels (7) along its length that could be used for multiple different purposes. See for example Figure 5. At their distal end, each internal channel terminates openly at the interior surface of the cup, and more preferably at the centre of the cup. In all embodiments of the invention, at least one internal channel is for extraction of fluid from the articular cavity. The extraction (not claimed) of fluid from the articular cavity by an internal channel can be made by application of a negative pressure at the proximal end. In a more preferred embodiment of the disclosure (not claimed), the extraction of fluid is limited to the confined volume located between the cup and the articular defect site, creating a negative pressure within the confined volume. In all embodiments, at least one internal channel is for transporting a therapeutic formulation from the proximal to the distal end for delivering it into a cartilage defect.

In all embodiments, at least one internal channel is for transmission of light (such as visible, infra-red or ultra-violet light) or other non-ionizing radiation (radiowaves or microwaves) that could be used to stimulate or initiate physicochemical processes within the cartilage defect site. Also in all embodiments, at least one internal channel is for passing an optical fibre to visualize the articular cavity and more specifically the cartilage defect.

The duration which the cup is maintained at the cartilage defect site may be adjusted. The duration which the cup is maintained after delivery of a therapeutic formulation is defined according to i) contact or diffusion of the therapeutic formulation within the cartilage defect site, or ii) progress of eventual physicochemical reactions taking place in the therapeutic formulation. The duration which the cup is maintained at the defect site is defined by the crosslinking kinetics of the therapeutic formulation.

After the delivery of therapeutic formulation, or whenever so required, the cup can be collapsed upon axial movement of the delivery arm relative to the external sleeve towards its proximal end.

In one embodiment, the external sleeve is made of a metallic material. In one embodiment, the external sleeve is made of a medical grade alloy. In an equally preferred embodiment, the external sleeve is made of a medical grade thermoplastic or thermoset material.

In one embodiment, the delivery arm is made of a thermoplastic material. In one embodiment, the delivery arm is made of polyolefin, polyester, or polyvinyl chloride. In one embodiment, the flexible arm is made of a thermoplastic elastomer (TPE). In one embodiment, the thermoplastic elastomer is selected from the following: TPA-polyamide TPE, TPC-copolyester TPE, TPO-olefinic TPE, TPS-styrenic TPE, TPU-urethane TPE, or TPV-thermoplastic rubber. In one embodiment, the delivery arm is made of a thermoset material. In one embodiment, the delivery arm is made of a thermoset elastomer selected from the following: siloxane, polyorganosiloxane or polysiloxane.

In one embodiment, the cup comprises a thermoplastic elastomer (TPE). In one embodiment, the thermoplastic elastomer is selected from the following: TPA-polyamide TPE, TPC-copolyester TPE, TPO-olefinic TPE, TPS-styrenic TPE, TPU-urethane TPE, or TPV-thermoplastic rubber. In one embodiment, the suction cup comprises a thermoset material. In one embodiment, the suction cup comprises a thermoset elastomer selected from the following: siloxane, polyorganosiloxane or polysiloxane.

The present disclosure provides a device for the delivery of therapeutic formulations aimed at treating or diagnosing articular cartilage defects and its method of use (not claimed). The defect may be located within a body cavity. The defect may be located within an articular cavity. The cartilage defect may be localized in the articular surface of a joint and its severity is classified between ICRS Grade 2 and ICRS Grade 4 B.

In a preferred embodiment of the disclosure (not claimed), an expansion fluid, which may be a liquid or gas, is used during arthroscopy. The liquid may be a sodium chloride solution, Ringer's solution, or a cell culture medium. In another embodiment of the disclosure (not claimed), the fluid is a gas such as sterile air comprising carbon dioxide in a concentration that is compatible with the tissue in question.

In one embodiment, the therapeutic formulation comprises at least one, or alternatively, any combination of components such as fluid vehicles, matrices, cells, therapeutic drugs, biomarkers, and biomolecules.

In one embodiment, the fluid vehicle is made of water buffered solution containing any of the following physiological relevant cations and anions such as Na⁺, Li⁺, K⁺, Ca²⁺, and Mg²⁺, Cl⁻, HCO₃⁻, HPO₄²⁻ and H₂PO₄⁻. In another preferred embodiment, the fluid vehicle is made of water buffered solution containing water soluble polymers or carbomers. In a preferred embodiment, the fluid vehicle is made of water buffered solution containing intra-articular lubricants that include polysaccharides or glycosaminoglycans. In a more preferred embodiment, the fluid vehicle is made of water buffered solution containing hyaluronic acid. In an equally preferred embodiment, the fluid vehicle is made of water buffered solution containing chondroitin sulphate.

In one embodiment, the matrix is made of a biocompatible biomaterial in a liquid, solution or suspension form, the biomaterial being of natural, synthetic or semi-synthetic origin. Acceptable diluents for preparing matrix solutions include, for example, sterile water for injection, saline solution and phosphate buffered saline.

In a more preferred embodiment, the matrix is a hydrogel. In one embodiment, the hydrogel comprises at least one of the following polymers or respective co-polymers such as: cyclodextrin, poly(p-dioxanone); poly(ethylene glycol), polyethylene glycol) methacrylate/dimethacrylate; poly(hydroxyethyl methacrylate); poly(acrylic acid); polyacrylamide; polyacrylonitrile; poly(butylene oxide); poly(ethylene glycol); poly(ethylene imine); poly(ethylene oxide); poly(ethyl methacrylate); propylene fumarate; poly(glucosylethyl methacrylate); poly(hydroxyethyl methacrylate); poly(hydroxypropyl methacrylamide); poly(methyl methacrylate); poly(N-isopropyl acrylamide); poly(N-vinyl pyrrolidone); polypropylene oxide); poly(vinyl alcohol); poly(vinyl acetate); or poly(vinyl amine), or combinations thereof. In another embodiment, the hydrogel comprises at least one of the following polymers, such as agarose, alginate, carrageenan, chitosan, gellan gum, hyaluronic acid, collagen, elastin, fibrin, gelatin, and silk fibroin, and combinations thereof. Such polymers may be used as such, or be chemically modified so as to optimise physicochemical and mechanical properties for particular therapeutic applications. In a preferred embodiment, the hydrogel exhibits adhesive properties towards living tissues without requirement for additional fixation aids. In a more preferred embodiment, the hydrogel exhibits adhesive properties towards connective tissues. In an even more preferred embodiment, the hydrogel exhibits adhesive properties towards cartilage tissue.

In one embodiment, cells are either from human or another animal. In a more preferred embodiment, cells consist of chondrocytes or chondrocyte progenitor cells that express genetic markers including but not limited to Sox9, COL2A1, ACAN and chondrogenic signature genes. In one embodiment, the cells are autologous and, in another embodiment, the cells are allogeneic. In a preferred embodiment, cells are co-delivered with a matrix. In an even more preferred embodiment, cells are co-delivered with a hydrogel.

In one embodiment, the therapeutic drug comprises: β-adrenergic agonists; anabolics; analgesics; anesthetics; anti-anginal agents; anti-arrythmics; antibacterial agents and antibiotics; anticoagulants and blood thinners; antidotes; antifungal agents; antihistaminics; anti-infectives; anti-inflammatory agents (steroidal and non-steroidal); antineoplastics; antipsoriatics; antiheumatics; antitumour agents; antiviral agents; bactericidins; bone densifiers; calcium channel blockers; carbonic anhydrase inhibitors; chemotherapeutics; coagulants; cystic fibrosis medications; dopamine receptor agonists and antagonists; estrogens; glucocorticoids; hemostatics; immunomodulators; immunosuppressants; multiple sclerosis medications; muscle relaxants; NMDA receptor antagonists; protease inhibitors; smooth muscle relaxants and stimulants; steroids; vasodilators; vasoprotectants or combinations thereof.

In one embodiment, the therapeutic formulation contains molecular biomarkers for diagnosing or determining the prognosis of cartilage diseases. In one embodiment, the therapeutic formulation contains molecular biomarkers for measuring collagen breakdown and turnover. In one embodiment, the therapeutic formulation contains molecular biomarkers for identification, and measurement of proteoglycan or glycosaminoglycan amount and location. In one embodiment, the therapeutic formulation contains molecular biomarkers for aggrecan, keratan sulfate, chondroitin sulfate, dermatan sulphate, heparin sulphate, heparin and / or hyaluronan.

In one embodiment, the biomolecule may be any of the following molecules: antibodies; amino acids; antigens; enzymes; hormones; peptides; polypeptides; proteins; polysaccharides; or combinations thereof.

When the device is held against an opposing surface, such as articular cartilage surface, to which the main axis of the device is orthogonal or oblique, the flexible cup can adapt itself to the opposing surface. In one embodiment, the interior surface of the cup is concave as to provide a better adjustment of the cup to the opposing convex cartilage surface in the joint. In another embodiment, the interior surface of the cup is convex as to provide a better adjustment of the cup to the opposing concave cartilage surface in the joint. In one embodiment, the size of the cup can be selected according to the size of the defect. In an equally preferred embodiment, the geometry of the cup can be selected according to the concave or convex radius of the opposing cartilage surface. In one embodiment, the internal surface of the cup is concave, the angle between the axis (24) and the edge (22) of the cup is between 0° and 90°. In another embodiment, the internal surface of the cup is convex, the angle between the axis (24) and the edge (22) of the cup is between 90° and 120°.

The present disclosure provides a method (not claimed) for arthroscopic implantation of therapeutic formulations into an articular cartilage defect.

The method, which does not form part of the claimed invention comprises:
1. Arthroscopically applying an expansion fluid to an articular cavity or surface containing the defect;
2. Determining the area, depth and location of the cartilage defect to be repaired;
3. Introducing the device into the articular cavity through the articular port;
4. Placing the device juxtaposed to the cartilage defect site inside the articular cavity;
5. Displacing the delivery arm and subsequently expanding the cup inside the articular cavity;
6. Covering the cartilage defect site with the expanded cup;
7. Sealing the defect site from the surrounding articular environment by using the cup, and create a confined volume at the cartilage defect site;
8. Optional: removing any fluid from the confined volume underneath the cup by applying negative pressure.
9. Delivering the therapeutic formulation by displacing it into the confined volume.
10. Optionally applying light or other radiation to the confined volume or therapeutic formulation in order to promote hydrogel formation.
11. Optionally visualizing the cartilage defect site.
12. Maintaining the cup at the cartilage defect site so as to promote contact or fixation of the therapeutic formulation to the cartilage defect site, and to allow any physicochemical reaction taking place in the therapeutic formulation to continue.
13. Removing the cup from the cartilage defect position and expose the cartilage defect site to the surrounding fluid.
14. Collapsing the cup by axial movement of the delivery arm towards its proximal end.
15. Removing the device.

The present disclosure provides a device and its method of use (not claimed) for delivering formulations into the articular joint, and more preferably to the defect of an articular cartilage.

One skilled in the art would understand the following description, as well as terminology used herein, as to best describe the disclosed subject matter, and embodiments chosen to do so are not intended to be exhaustive or to limit the disclosure to the form disclosed. The invention is defined in the claims.

In an embodiment, the device is intended to be used during an arthroscopy procedure to deliver a therapeutic formulation into a cartilage defect by an arthroscopic port. The device allows a continuous connection between the cartilage defect cavity and an aseptically controlled environment outside the body. The device and all of its components have a distal end and a proximal end. In an embodiment, the distal end is intended to be used inside the articular cavity while the proximal end faces the surgeon and provides access to the interior of the device. In a preferred embodiment, the device exhibits enough flexibility to allow easy entering and manoeuvring within the articular cavity during the arthroscopy procedure.

In an embodiment, the device was manufactured using materials suitable for medical device manufacturing. The external sleeve was manufactured using polyetherimide (PEI) thermoplastic, for example a ULTEM^{®} rod 1000 series (Sabic). The external sleeve has an outer radius of 10 mm, a wall thickness 0.6 mm and, a length of 140 mm. The internal sleeve was manufactured using polyetherimide (PEI) thermoplastic, for example a ULTEM^{®} rod 1000 series (Sabic). The internal sleeve has an outer radius of 8 mm, a wall thickness of 0.3 mm and, a length of 160 mm. The delivery arm and cup were manufactured from polysiloxane materials (Silastic, Dupont). The delivery arm and cup were moulded from machined master part. The delivery arm has an outer radius of 7 mm and a length of 200 mm. The delivery arm has 2 channels, each with a 2 mm diameter. After manufacturing, the parts were washed with a mild washing detergent (Softaskin, B.Braun), rinsed in ultrapure water (Milli-Q, Millipore), wrapped for sterilization and autoclaved (132 ºC, 210 kPa, 50 min). After sterilization, all sterilized parts were unpacked inside a flow chamber and manually assembled. Thereafter, the device is wrapped and subjected to a new cycle of sterilization by ethylene oxide (EtO) (80% EtO, 20% CO₂) at 45 ºC ± 5 ºC, 1400 mbar for a duration of 240 min.

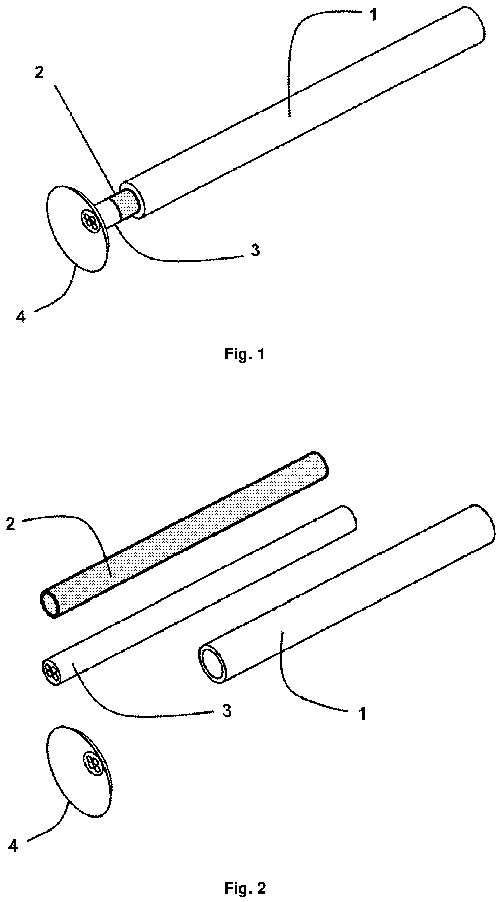

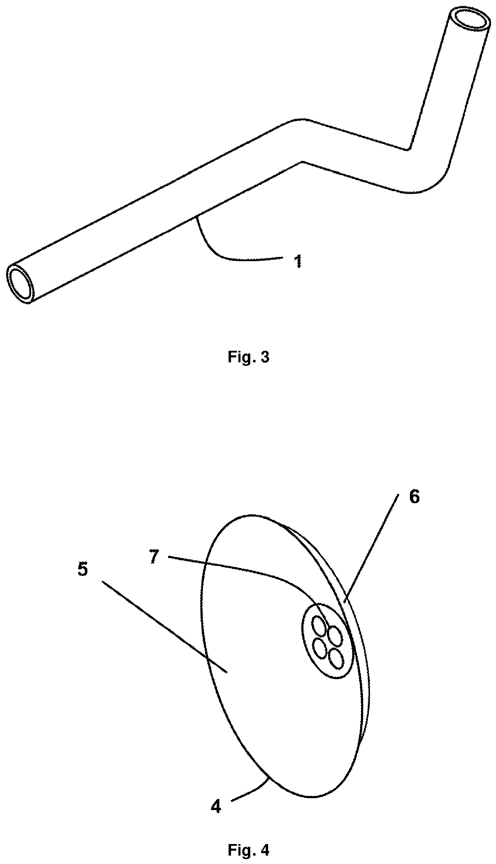

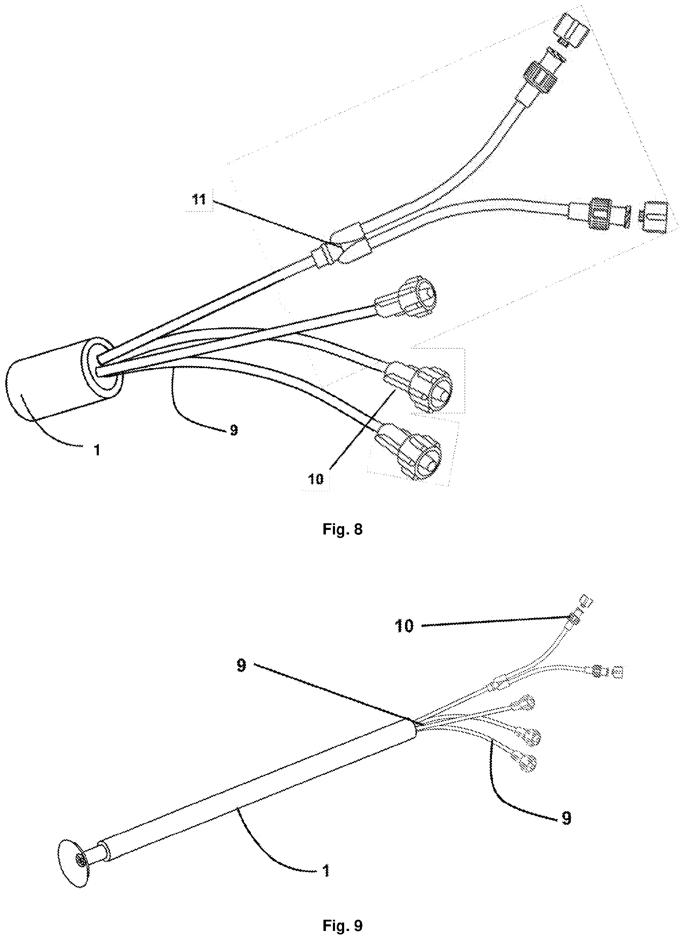

In an embodiment, Figure 1 shows the distal section of the device wherein (1) represents the external sleeve, (2) represents the internal sleeve, (3) represents the delivery arm and (4) represents the cup. Figure 2 shows an exploded view of the device featuring its main components wherein (1) represents the external sleeve, (2) represents the internal sleeve, (3) represents the delivery arm and (4) represents the cup. Figure 3 shows a schematic representation of the external sleeve (1) of the device in a bended configuration. Figure 4 shows a schematic representation of the cup (4) in its expanded configuration wherein (5) represents the interior surface, (6) represents the exterior surface and (7) represents the internal channels. There are four internal channels. Figure 5 shows a schematic representation of the delivery arm (3) featuring four internal channels (7). Figure 6 shows a schematic representation of the cross-section of the delivery arm (3) showing one internal channel (7) and its displaceable plunger (8). Figure 7 shows a schematic representation of the gradual expansion of the cup (4) during axial displacement of the delivery arm (3) towards the distal end within the external sleeve (1) wherein: (1) represents the external sleeve, (4) represents the cup and, (5) represents the interior surface. Figure 8 shows a schematic representation of the proximal section of the device wherein (3) represents the delivery arm, (9) represents the connection tubes, (10) represents the connection ports and, (11) represents the double connectors. The connection tubes are used for delivery of therapeutic formulations, administration or removal of fluids, transmission of radiation, and visualization of treatment area. Figure 9 shows a Schematic representation of the device wherein (1) represents the external sleeve, (9) represents the connection tubes and, (10) represents the connection ports. The connection tubes are for delivery of therapeutic formulations, administration or removal of fluids and, transmission of radiation, and visualization of treatment area.

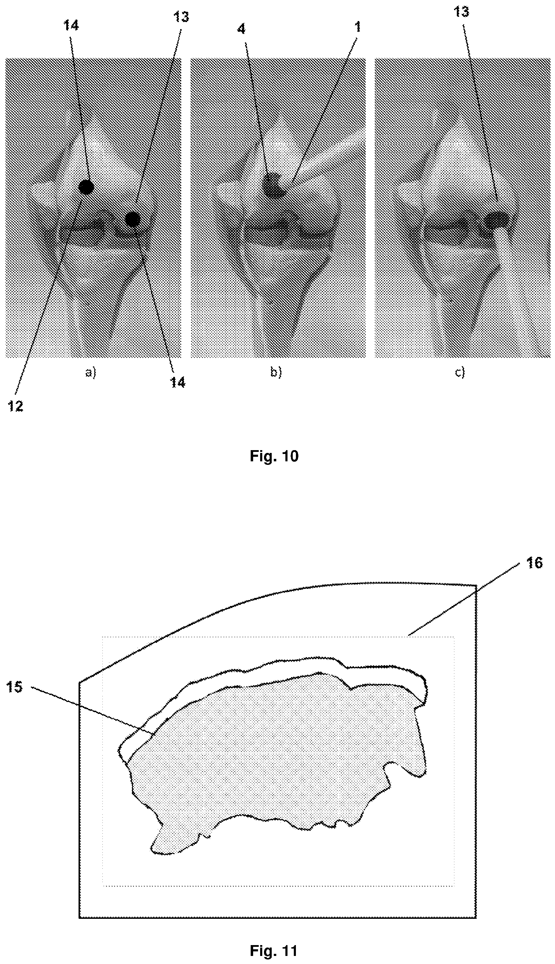

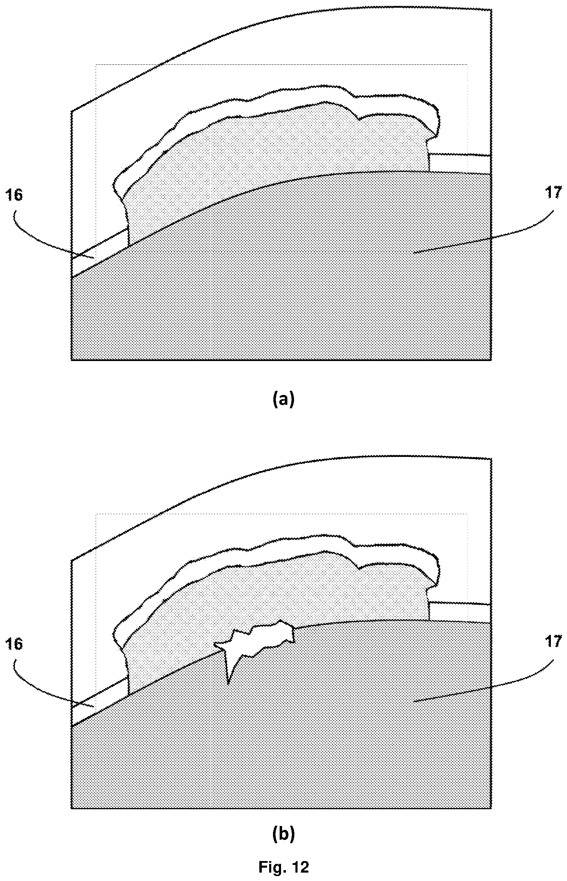

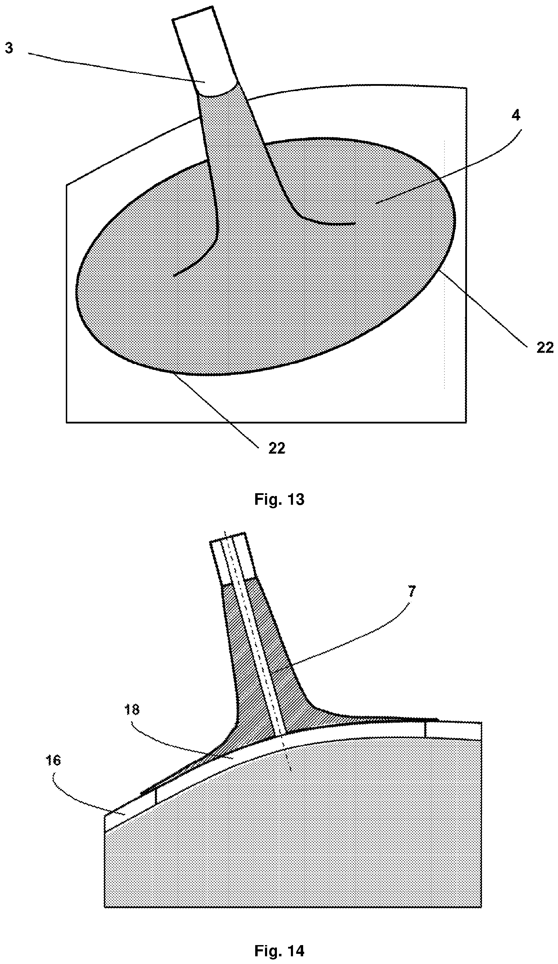

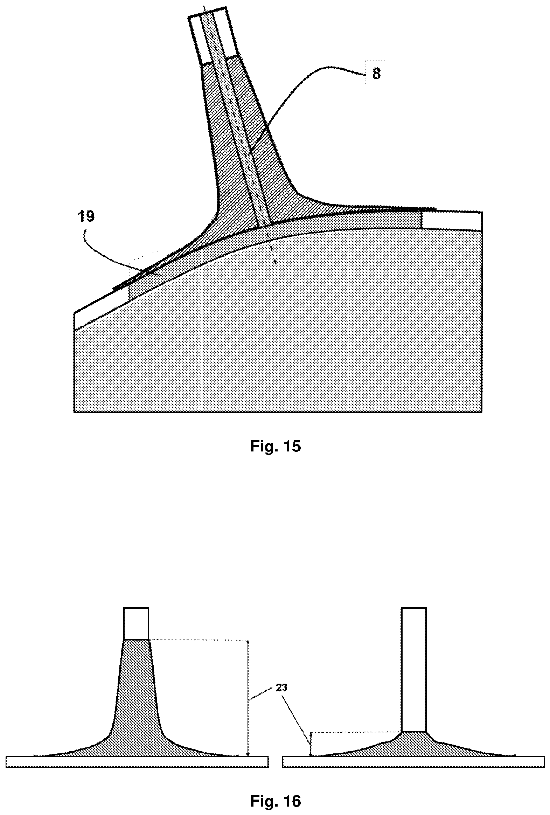

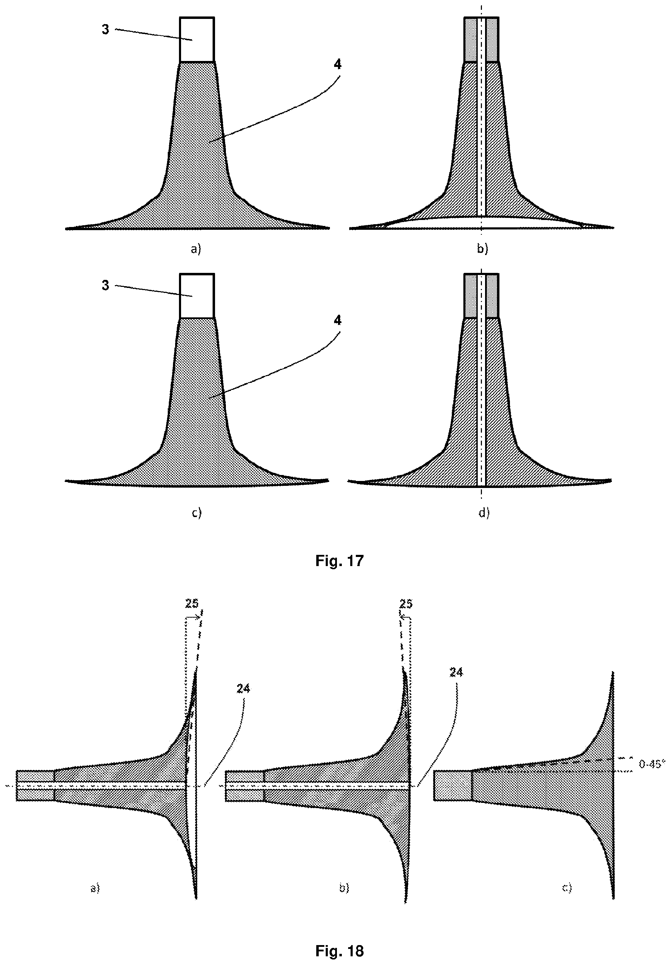

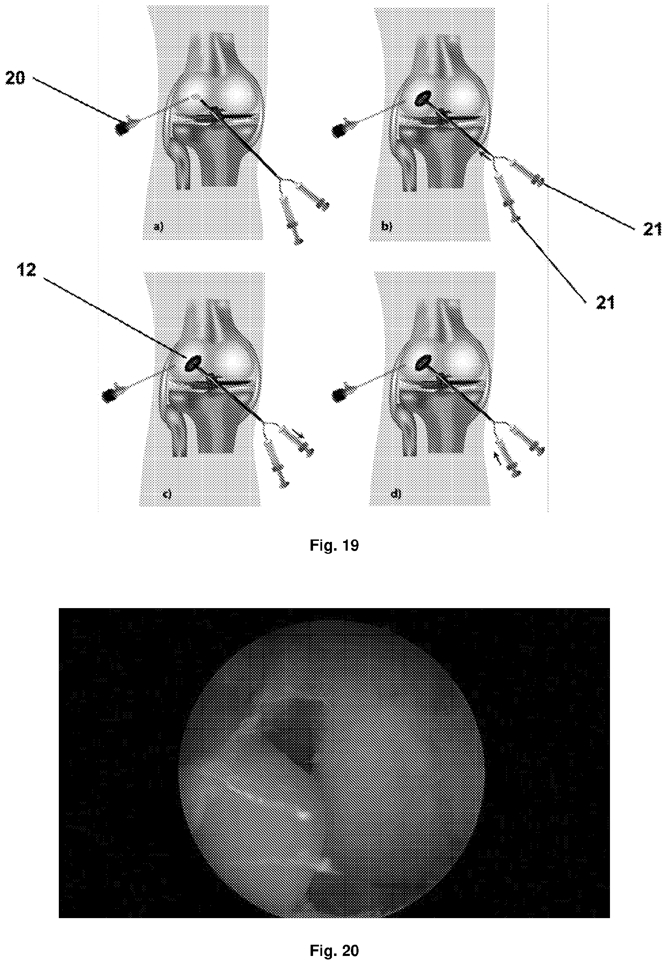

Figure 10 shows a Picture of a three-dimensional model of an articular knee joint. Figure 10a) shows the articular knee joint with two cartilage defect sites (14) schematically represented by dark circles located on the lateral condyle (12) and the medial condyle (13). Figure 10b) shows coverage of the lateral condyle cartilage defect site by the expanded cup (4). Figure 10c) shows coverage of the medial condyle cartilage defect site by the expanded cup (4). The patella ligament has been moved laterally to facilitate visualization of the lateral and medial condyles. Figure 11 shows a Schematic representation of articular cartilage defect (15) surrounded by cartilage tissue (16). Figure 12 shows a Schematic representation of a cross-section of an articular cartilage defect (15) and its surrounding cartilage (16). In Figure 12(a) the exposed subchondral bone (17) in exposed. In Figure 12(b) the subchondral bone (17) is exposed and there is cavitation in the subchondral bone. Figure 13 shows a Schematic representation of the perspective view of the cup covering an articular cartilage defect wherein: (3) represents the delivery arm, (4) represents the cup and, (22) represents the edge of the cup. Figure 14 shows a Schematic representation of the cross-section of the cup covering an articular cartilage defect and the cartilage defect volume wherein: (7) represents the internal channel, (16) represents the surrounding articular cartilage and, (18) represents the cartilage defect volume. Figure 15 shows a Schematic representation of the cross-section of the cup covering an articular cartilage defect after filling of cartilage defect volume with therapeutic formulation wherein: (8) represents the plunger and (19) represents the therapeutic formulation. Figure 16 shows a Schematic representation of the side view of the cup in two different heights wherein (23) represents the height of the cup. Figure 17 shows a Schematic representation of the side view and cross-section view of the with concave and convex interior surface. Figure 17a) shows the side view of cup with concave interior surface. Figure 17b) shows the cross-section view of cup with concave interior surface. Figure 17c) shows the side view of cup with convex interior surface. Figure 17d) shows the cross-section view of cup with convex interior surface. Figure 18 shows a Schematic representation of the cup and the angle between the axis and the edge of the cup wherein (24) represents the axis of the cup and (25) represents the angle between the axis of the cup and the edge of the cup. Figure 18a) shows the cross-section of the cup with concave interior surface. Figure 18b) shows the cross-section view of the cup with convex interior surface. Figure 18c) shows that the angle between the axis of the cup and the edge of the cup is between 0° - 45°. Figure 19 shows a Schematic representation of the arthroscopic implantation of therapeutic formulations into an articular cartilage defect wherein: (12) represents the lateral condyle, (20) represents the endoscope and (21) represents the syringe. Figure 19a) shows the placement of the delivery device, with visual aid from the endoscope, juxtaposed to the cartilage defect site inside the articular cavity. Figure 19b) shows the displacement of the delivery arm, the expansion of the cup inside the articular cavity, followed by covering of the cartilage defect site by the expanded cup. Figure 19c) shows the removal of fluid, through a syringe (21), from the confined volume underneath the expanded cup. Figure 19d) shows the delivery of a therapeutic formulation, through a syringe (21,) into the defect site.

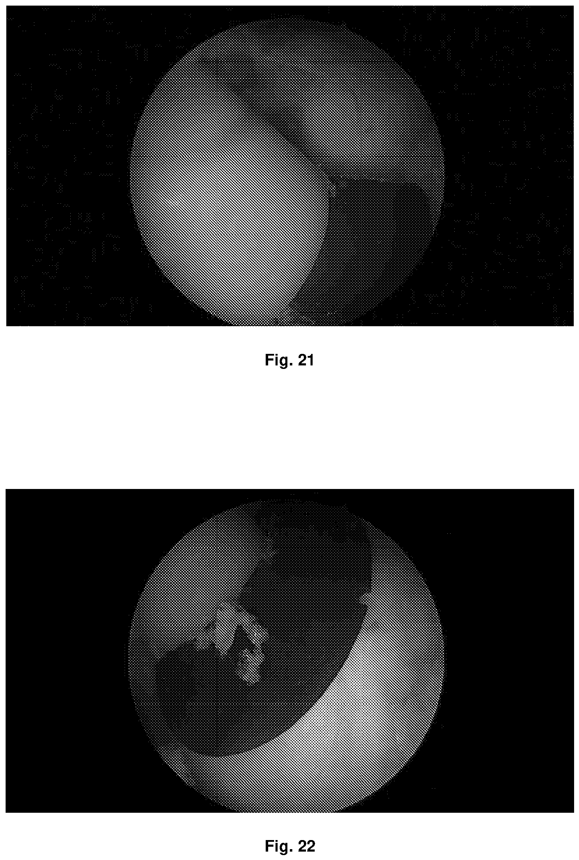

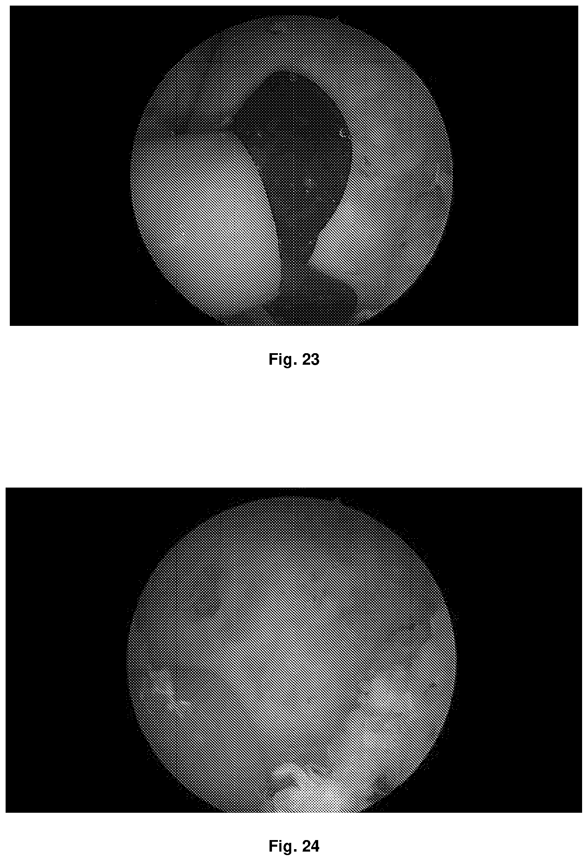

Figure 20 shows an Arthroscopic image of the arthroscopic implantation of therapeutic formulations into an articular cartilage defect in a cadaveric model: device being juxtaposed to a cartilage defect. Figure 21 shows an Arthroscopic image of the arthroscopic implantation of therapeutic formulations into an articular cartilage defect in a cadaveric model: expansion of the cup. Figure 22 shows an Arthroscopic image of the arthroscopic implantation of therapeutic formulations into an articular cartilage defect in a cadaveric model: covering of the cartilage defect site by the expanded cup and subsequently delivering a therapeutic formulation to the cartilage defect site. Figure 23 shows an Arthroscopic image of the arthroscopic implantation of therapeutic formulations into an articular cartilage defect in a cadaveric model: removal of the cup. Figure 24 shows an Arthroscopic image of the arthroscopic implantation of therapeutic formulations into an articular cartilage defect in a cadaveric model: therapeutic formulation delivered to the site.

Furthermore, in what concerns the figures, (1) represents an external sleeve, (2) an internal sleeve, (3) a delivery arm, (4) a cup, (5) the interior surface, (6) the exterior surface, (7) the internal channel, (8) a plunger, (9) a connection tube, (10) a double connector, (11) a lateral condyle, (12) a medial condyle, (13) the cartilage defect location, (14) a cartilage defect, (15) the articular cartilage surrounding the defect, (16) the subchondral bone, (17) the cartilage defect volume, (18) a therapeutic formulation, (19) an endoscope, (20) a syringe, (21) the edge of the cup, (22) the height of the cup, (23) the axis of the cup and, (24) the angle between the axis and the edge of the cup.

In an embodiment, the following pertains to a knee arthroscopy. A human cadaveric knee used for demonstrating the efficacy and method of using the device. The cadaveric knee was fixed to a support table. The knee was positioned in a 90º flexion with the distal end of the leg in suspension. Two ports were open on the knee with a surgical blade: one antero-lateral port and one antero-medial port. The procedure was carried out using the following arthroscopy instruments: an arthroscope tower fitted with a 30º wide angle telescope that has an integrated light source (Karl Storz) and is connected to a monitor screen; a double roller pump (Arthropump, Karl Storz) for articular cavity irrigation. Ringer's solution (Sigma Aldrich) was used as fluid for irrigating the cavity during the arthoscopic procedure. The intra-articular irrigation of the knee (100 mL/min) was performed during distension of the knee joint (average pressure 70 mmHg). The arthroscope was introduced into the knee cavity for intra-articular knee inspection. A 5 mm diameter defect was induced in the femoral condyle with a drill using a motorized drill.

The following pertains to device configuration and therapeutic formulation. The device was used by adopting a configuration described in Figure 19 shows how the device was configured in order to demonstrate the function and performance of the device. Two connection tubes are independently connected to two distinct internal channels. Two syringes (one empty and one containing a therapeutic formulation) were connected to the connection tubes of the delivery arm via Luer connectors. A therapeutic formulation was prepared and inserted into one of the syringes that is connected to a connection tube via a Luer connector. The therapeutic formulation (total volume of 1 mL) comprises methacrylated gellan gum hydrogel (2 % w/V) and a suspension of 2×10⁶ cells/mL (human mesenchymal stem cells). Phosphate buffer saline (Gibco) with calcium and magnesium was used as a fluid vehicle.

The following pertains to therapeutic delivery. After the induction of the defect in the knee, the device was inserted into the articular cavity . The device was inserted through the antero-medial port. Figure 20 shows the device placed juxtaposed to the cartilage defect site inside the articular cavity. This was done with the aid of an endoscope. Figure 21 shows the delivery arm displaced to allow expansion of the cup inside the articular cavity. This is done so that the cup can cover the defect site. Figure 22 shows the device firmly positioned over the defect site thus effectively sealing and isolating the defect site from the surrounding articular environment by the cup. Figure 19 c) shows 0.2 mL of fluid being removed from the confined volume underneath the cup. This is done by withdrawing the plunger of the empty syringe which then creates an area of negative pressure under the cup. Figure 19 d) shows the therapeutic formulation being delivered into the articular defect site by pushing the plunger forward. In order to ensure that there is sufficient time for fixation of the therapeutic formulation on the defect site and for crosslinking of the matrix by divalent cations, the cup was was kept on top of the cartilage defect site for approximately 5 minutes. Figure 23 shows the cup being subsequently removed and the cartilage defect site is exposed to the surrounding articular fluid. The cup was collapsed by axial movement of the delivery arm relatively to the external sleeve towards its proximal end, and the device removed - Figure 24. An endoscope was used during the arthroscopic procedure to allow optical observation of the process and to confirm that the therapeutic formulation has fixed onto the cartilage defect.

Further areas of applicability are apparent from the description provided herein. It should be understood that the description and specific examples are intended for purposes of illustration only and are not intended to limit the scope of the present disclosure.

The term "comprising" whenever used in this document is intended to indicate the presence of stated features, integers, steps, components, but not to preclude the presence or addition of one or more other features, integers, steps, components or groups thereof. The above described embodiments are combinable. The following claims define the invention.

### References

Basad, E. et al., 2010. Matrix-induced autologous chondrocyte implantation versus microfracture in the treatment of cartilage defects of the knee: A 2-year randomised study. Knee Surgery, Sports Traumatology, Arthroscopy, 18(4), pp.519-527.
Benthien, J.P. & Behrens, P., 2011. The treatment of chondral and osteochondral defects of the knee with autologous matrix-induced chondrogenesis (AMIC): Method description and recent developments. Knee Surgery, Sports Traumatology, Arthroscopy, 19(8), pp.1316-1319.
Brittberg, M. et al., 1994. Treatment of deep cartilage defects in the knee with autologous chondrocyte transplantation. The New England journal of medicine, 331(14), pp.889-95.
Carr, A.J. et al., 2014. Advances in arthroscopy-indications and therapeutic applications. Nature Reviews Rheumatology, 11(2), pp.77-85.
Harris, J.D. et al., 2011. Failures, re-operations, and complications after autologous chondrocyte implantation - a systematic review. Osteoarthritis and Cartilage, 19(7), pp.779-791.
Ibarra, C. et al., 2014. Follow-up of a new arthroscopic technique for implantation of matrixencapsulated autologous chondrocytes in the knee. Arthroscopy - Journal of Arthroscopic and Related Surgery, 30(6), pp.715-723.
Lum, L., Elisse, J. & Elisseeff, J., 2003. Injectable hydrogels for cartilage tissue engineering. Topics in tissue engineering, pp.1-25.
Mithoefer, K. et al., 2005. The Microfracture Technique for the Treatment of Articular Cartilage Defects in the Knee. J Bone JointSurg Am, 87(9), pp.1911-1920.
Peterson, L. et al., 2000. Two- to 9-year outcome after autologous chondrocyte transplantation of the knee. Clinical orthopaedics and related research, (374), pp.212-34.
Vascellari, A. et al., 2014. Implantation of matrix-induced autologous chondrocyte (MACI) grafts using carbon dioxide insufflation arthroscopy. Knee Surgery, Sports Traumatology, Arthroscopy, 22(1), pp.219-225.
Widuchowski, W., Widuchowski, J. & Trzaska, T., 2007. Articular cartilage defects: Study of 25,124 knee arthroscopies. Knee, 14(3), pp.177-182.

## Claims

1. An arthroscopic surgery device for delivering a therapeutic formulation into a cartilage of an articular joint, wherein the device comprises:
a flexible tubular delivery arm (3), comprising more than one internal channels (7) for carrying
the therapeutic formulation wherein the more than one internal channels (7) comprise a
channel for extracting fluid from an articular cavity, a channel for carrying the therapeutic formulation, a channel for transmitting radiation, and a channel for passing an optical fibre for visualization;
a flexible external sleeve (1) enveloping the delivery arm lengthwise;
a pliable cup (4) attached to an end of the delivery arm (3), for covering a cartilage part where the therapeutic formulation is to be delivered;
arranged such that by outwards axial displacement of the delivery arm (3) in respect to the external sleeve (1), the cup moves from inside to outside of the external sleeve (1), expanding the cup (4).

2. The arthroscopic surgery device according to the previous claim further comprising a flexible internal sleeve (2) between the delivery arm (3) and the external sleeve (1), such that the flexible internal sleeve (2) envelops the delivery arm lengthwise; preferably the internal sleeve (2) is attached to the delivery arm (3), in particular such that they move in tandem.

3. The arthroscopic surgery device according to any of the previous claims wherein one or more internal channels (7) comprises a plunger (8) for pushing the therapeutic formulation to the cartilage part where the therapeutic formulation is to be delivered; and/or one or more internal channels (7) comprises a plunger (8) for pulling fluid from the cartilage part.

4. The arthroscopic surgery device according to any of the previous claims for extracting fluid from an articular cavity.

5. The arthroscopic surgery device according to any of the previous claims wherein the cup (4) is trumpet-shaped, and/or conical and flexible.

6. The arthroscopic surgery device according to any of the previous claims wherein the cup (4) is shaped concavely to cover the cartilage part where the therapeutic formulation is to be delivered, and/or comprises an edge (22) for covering the cartilage part, wherein said edge (22) is planar.

7. The arthroscopic surgery device according to any of the claims 1-5 wherein the cup (4) is shaped to project convexly when covering the cartilage part where the therapeutic formulation is to be delivered.

8. The arthroscopic surgery device according to any of the previous claims wherein the external sleeve (1), the internal sleeve (2) and the delivery arm (3) are concentric tubes.

9. The arthroscopic surgery device according to any of the previous claims wherein the delivery arm (3) is more flexible than the external sleeve (1).

10. The arthroscopic surgery device according to any of the previous claims wherein one or more internal channels (7) comprises an optical fibre for transmitting light to articular joint; and/or one or more internal channels (7) is arranged to carry a fluid, in particular a therapeutic formulation fluid or an expansion fluid, further in particular a gas or a liquid; and/or one or more internal channels (7) comprise a therapeutic formulation, in particular a liquid or hydrogel therapeutic formulation, further in particular the liquid therapeutic formulation being a solution or a suspension.

11. The arthroscopic surgery device according to any of the previous claims wherein the therapeutic formulation comprises a fluid vehicle, a biocompatible matrix, cellular material for cell therapy, therapeutic drug, a biomarker, or mixtures thereof.

12. The arthroscopic surgery device according to any of the previous claims wherein the delivery arm (3), the internal sleeve (2), or the delivery arm (3) and the internal sleeve (2) are made of a thermoplastic material.

13. The arthroscopic surgery device according to any of the previous claims comprising one or more connection tubes (9), each for the one or more internal channels (7), and/or one or more connection ports (10), each for the one or more connection tubes (9).

14. The arthroscopic surgery device according to any of the previous claims wherein the external sleeve (1) is made of a metallic material, more preferably a medical grade alloy, the external sleeve (1) is made of a thermoplastic material, or the external sleeve (1) is made of a thermoset material; preferably the external sleeve (1) is metallic.

15. A kit of parts comprising:
the arthroscopic surgery device according to any of the previous claims; and
the therapeutic formulation,
wherein the therapeutic formulation comprises:
a therapeutic agent and a fluid vehicle for said therapeutic agent, wherein the fluid vehicle is water solution containing any of the following physiologically relevant ions Na⁺, Li⁺, K⁺, Ca²⁺, and Mg²⁺, Cl⁻, HCO₃⁻, HPCO₄²⁻ and H₂PO₄⁻; a matrix, wherein the matrix comprises a biocompatible biomaterial in a liquid, solution or suspension form, or a hydrogel; cells, wherein the cells are cartilage forming cells; a therapeutic drug; a biomarker; a biomolecule; or combinations thereof.

## Patentansprüche

1. Eine Vorrichtung für arthroskopische Chirurgie zur Verabreichung einer therapeutischen Formulierung in einen Knorpel eines Gelenks, wobei die Vorrichtung umfasst:
einen flexiblen, röhrenförmigen Zuführungsarm (3), umfassend mehr als einen inneren Kanäle (7) zum Transport der therapeutischen Formulierung, wobei die mehr als einen inneren Kanäle (7) umfassen einen Kanal zum Extrahieren von Flüssigkeit aus einer Gelenkhöhle, einen Kanal zum Transport der therapeutischen Formulierung, einen Kanal zum Übertragen von Strahlung und einen Kanal zum Durchführen einer optischen Faser zur Visualisierung;
eine flexible äußere Hülse (1), die den Zuführungsarm in Längsrichtung umschließt;
eine biegsame Schale (4), die an einem Ende des Zuführungsarms (3) angebracht ist, um einen Knorpelteil abzudecken, an dem die therapeutische Formulierung abgegeben werden soll;
die so angeordnet ist, dass durch eine nach außen gerichtete axiale Verschiebung des Zuführungsarms (3) in Bezug auf die äußere Hülse (1) die Schale sich vom Innern der äußeren Hülse (1) nach außen bewegt und die Schale (4) dabei gedehnt wird.

2. Die Vorrichtung für arthroskopische Chirurgie nach dem vorangehenden Anspruch, ferner umfassend eine flexible innere Hülse (2) zwischen dem Zuführungsarm (3) und der äußeren Hülse (1), so dass die flexible innere Hülse (2) den Zuführungsarm in Längsrichtung umhüllt; wobei die innere Hülse (2) bevorzugt am Zuführungsarm (3) befestigt ist, insbesondere so, dass sie sich im Tandem bewegen.

3. Die Vorrichtung für arthroskopische Chirurgie nach einem der vorangehenden Ansprüche, wobei einer oder mehrere innere Kanäle (7) einen Kolben (8) umfassen, um die therapeutische Formulierung zu dem Knorpelteil zu drücken, an dem die therapeutische Formulierung abgegeben werden soll; und/oder einer oder mehrere innere Kanäle (7), umfassend einen Kolben (8), um Flüssigkeit aus dem Knorpelteil zu saugen.

4. Die Vorrichtung für arthroskopische Chirurgie nach einem der vorangehenden Ansprüche zum Absaugen von Flüssigkeit aus einer Gelenkhöhle.

5. Die Vorrichtung für arthroskopische Chirurgie nach einem der vorangehenden Ansprüche, wobei die Schale (4) trompetenförmig und/oder konisch und flexibel ist.

6. Die Vorrichtung für arthroskopische Chirurgie nach einem der vorangehenden Ansprüche, wobei die Schale (4) konkav geformt ist, um den Knorpelteil zu bedecken, an dem die therapeutische Formulierung abgegeben werden soll, und/oder einen Rand (22) zum Bedecken des Knorpelteils aufweist, wobei der genannte Rand (22) plan ist.

7. Die Vorrichtung für arthroskopische Chirurgie nach einem der Ansprüche 1-5, wobei die Schale (4) so geformt ist, dass sie konvex vorsteht, wenn sie den Knorpelteil bedeckt, an dem die therapeutische Formulierung abgegeben werden soll.

8. Die Vorrichtung für arthroskopische Chirurgie nach einem der vorangehenden Ansprüche, wobei die äußere Hülse (1), die innere Hülse (2) und der Zuführungsarm (3) konzentrische Rohre sind.

9. Die Vorrichtung für arthroskopische Chirurgie nach einem der vorangehenden Ansprüche, wobei der Zuführungsarm (3) flexibler ist als die äußere Hülse (1).

10. Die Vorrichtung für arthroskopische Chirurgie nach einem der vorangehenden Ansprüche, wobei einer oder mehrere innere Kanäle (7) eine optische Faser zur Übertragung von Licht auf das Gelenk umfassen; und/oder einer oder mehrere innere Kanäle (7) so angeordnet sind, dass sie ein Fluid, insbesondere eine flüssige therapeutische Formulierung oder ein Expansionsfluid, ferner insbesondere ein Gas oder eine Flüssigkeit, transportieren; und/oder einer oder mehrere innere Kanäle (7), umfassend eine therapeutische Formulierung, insbesondere eine therapeutische Formulierung in Form einer Flüssigkeit oder eines Hydrogels, weiter insbesondere die flüssige therapeutische Formulierung eine Lösung oder eine Suspension ist.

11. Die Vorrichtung für arthroskopische Chirurgie nach einem der vorangehenden Ansprüche, wobei die therapeutische Formulierung ein flüssiges Mittel, eine biokompatible Matrix, zelluläres Material für die Zelltherapie, ein therapeutisches Arzneimittel, einen Biomarker oder Mischungen davon umfasst.

12. Die Vorrichtung für arthroskopische Chirurgie nach einem der vorangehenden Ansprüche, wobei der Zuführungsarm (3), die innere Hülse (2) oder der Zuführungsarm (3) und die innere Hülse (2) aus einem thermoplastischen Material hergestellt sind.

13. Die Vorrichtung für arthroskopische Chirurgie nach einem der vorangehenden Ansprüche, umfassend einen oder mehrere Verbindungsschläuche (9), jeweils für den einen oder die mehreren internen Kanäle (7), und/oder einen oder mehrere Anschlüsse (10), jeweils für den einen oder die mehreren Verbindungsschläuche (9).

14. Die Vorrichtung für arthroskopische Chirurgie nach einem der vorangehenden Ansprüche, wobei die äußere Hülse (1) aus einem metallischen Material, bevorzugt einer für medizinische Zwecke geeigneten Metalllegierung gefertigt ist, die äußere Hülse (1) aus einem thermoplastischen Material gefertigt ist oder die äußere Hülse (1) aus einem duroplastischen Material gefertigt ist; wobei die äußere Hülse (1) bevorzugt metallisch ist.

15. Ein Teilesatz bestehend aus:
die Vorrichtung für arthroskopische Chirurgie nach einem der vorangehenden Ansprüche, und
die therapeutische Formulierung,
wobei die therapeutische Formulierung umfasst:
ein therapeutisches Mittel und ein flüssiges Mittel für das genannte therapeutische Mittel, wobei das flüssige Mittel eine Wasserlösung ist, die eines der folgenden physiologisch relevanten Ionen enthält: Na⁺, Li⁺, K⁺, Ca²⁺, und Mg²⁺, Cl⁻, HCO₃⁻; HPO₄²⁻ und H₂PO₄⁻; eine Matrix, wobei die Matrix ein biokompatibles Biomaterial in Form einer Flüssigkeit, Lösung oder Suspension oder eines Hydrogels umfasst; Zellen, wobei die Zellen knorpelbildende Zellen sind; ein therapeutisches Arzneimittel; einen Biomarker; ein Biomolekül; oder Kombinationen davon.

## Revendications

1. Un dispositif de chirurgie arthroscopique pour l'administration d'une formulation thérapeutique dans un cartilage d'une articulation, dans lequel le dispositif comprend :
un bras d'administration tubulaire flexible (3) comprenant plusieurs canaux internes (7) pour transporter la formulation thérapeutique dans lequel les plusieurs canaux internes (7) comprennent un canal pour extraire du fluide d'une cavité articulaire, un canal pour transporter la formulation thérapeutique, un canal pour transmettre de la radiation, et un canal pour passer une fibre optique destinée à la visualisation ;
un manchon externe flexible (1) enveloppant le bras d'administration dans le sens de la longueur ;
une coupelle pliable (4) fixée à une extrémité du bras d'administration (3), pour couvrir une partie de cartilage où la formulation thérapeutique doit être administrée ;
arrangé tel que à travers le déplacement axial vers l'extérieur du bras d'administration (3) par rapport au manchon externe (1), la coupelle se meuvent de l'intérieur vers l'extérieur du manchon externe (1), dilatant la coupelle (4).

2. Le dispositif de chirurgie arthroscopique selon la revendication précédente comprenant également un manchon interne flexible (2) entre le bras d'administration (3) et le manchon externe (1), tel que le manchon interne flexible (2) enveloppe le bras d'administration dans le sens de la longueur ; le manchon interne (2) étant préférablement fixé au bras d'administration (3), en particulier tel qu'ils se meuvent en tandem.

3. Le dispositif de chirurgie arthroscopique selon l'une quelconque des revendications précédentes dans lequel un ou plusieurs des canaux internes (7) comprennent un piston (8) pour pousser la formulation thérapeutique vers la partie de cartilage où la formulation thérapeutique doit être administrée ; et/ou un ou plusieurs canaux internes (7) comprennent un piston (8) pour retirer du fluide de la partie de cartilage.

4. Le dispositif de chirurgie arthroscopique selon l'une quelconque des revendications précédentes pour extraire du fluide d'une cavité articulaire.

5. Le dispositif de chirurgie arthroscopique selon l'une quelconque des revendications précédentes dans lequel la coupelle (4) est en forme de trompette, et/ou conique et flexible.

6. Le dispositif de chirurgie arthroscopique selon l'une quelconque des revendications précédentes dans lequel la coupelle (4) a une forme concave pour couvrir la partie de cartilage où la formulation thérapeutique doit être administrée, et/ou comprend un bord (22) pour couvrir la partie de cartilage, dans laquelle ledit bord (22) est planaire.

7. Le dispositif de chirurgie arthroscopique selon l'une quelconque des revendications 1-5 dans lequel la coupelle (4) est formée de manière à être projetée de façon convexe lorsqu'elle couvre la partie de cartilage où la formulation thérapeutique doit être administrée.

8. Le dispositif de chirurgie arthroscopique selon l'une quelconque des revendications précédentes dans lequel le manchon externe (1), le manchon interne (2) et le bras d'administration (3) sont des tubes concentriques.

9. Le dispositif de chirurgie arthroscopique selon l'une quelconque des revendications précédentes dans lequel le bras d'administration (3) est plus flexible que le manchon externe (1).

10. Le dispositif de chirurgie arthroscopique selon l'une quelconque des revendications précédentes dans lequel un ou plusieurs canaux internes (7) comprennent une fibre optique pour transmettre de la lumière à une articulation ; et/ou un ou plusieurs canaux internes (7) sont arrangés de manière à transporter un fluide, en particulier un fluide de formulation thérapeutique ou un fluide d'expansion, plus particulièrement un gaz ou un liquide ; et/ou un ou plusieurs canaux internes (7) comprennent une formulation thérapeutique, en particulier une formulation thérapeutique de liquide ou hydrogel, la formulation thérapeutique de liquide étant plus particulièrement une solution ou une suspension.

11. Le dispositif de chirurgie arthroscopique selon l'une quelconque des revendications précédentes dans lequel la formulation thérapeutique comprend un véhicule de fluide, une matrice biocompatible, un matériau cellulaire destiné à la thérapie cellulaire, un médicament thérapeutique, un biomarqueur, ou des mélanges de ceux-ci.

12. Le dispositif de chirurgie arthroscopique selon l'une quelconque des revendications précédentes dans lequel le bras d'administration (3), le manchon interne (2), ou le bras d'administration (3) et le manchon interne (2) sont faits d'un matériau thermoplastique.

13. Le dispositif de chirurgie arthroscopique selon l'une quelconque des revendications précédentes comprenant un ou plusieurs tubes de raccord (9), chacun pour le ou les canaux internes (7), et/ou un ou plusieurs ports de raccord (10), chacun pour le ou les tubes de raccord (9).

14. Le dispositif de chirurgie arthroscopique selon l'une quelconque des revendications précédentes dans lequel le manchon externe (1) est fait d'un matériau métallique, plus préférablement d'un alliage de qualité médicale, le manchon externe (1) est fait d'un matériau thermoplastique, ou le manchon externe (1) est fait d'un matériau thermodurci ; le manchon externe (1) étant préférablement métallique.

15. Un kit de pièces comprenant :
le dispositif de chirurgie arthroscopique selon l'une quelconque des revendications précédentes ; et
la formulation thérapeutique,
dans lequel la formulation thérapeutique comprend :
un agent thérapeutique et un véhicule de fluide pour ledit agent thérapeutique, dans lequel le véhicule de fluide est une solution aqueuse contenant l'un quelconque des ions physiologiquement pertinents suivants : Na⁺, Li⁺, K⁺, Ca²⁺ et Mg²⁺, Cl⁻, HCO₃⁻, HPO₄²⁻ et H₂PO₄⁻ ; une matrice, dans laquelle la matrice comprend un biomatériau biocompatible sous forme liquide, de solution ou de suspension, ou un hydrogel ; des cellules, dans lesquelles les cellules sont des cellules de formation de cartilage ; un médicament thérapeutique ; un biomarqueur ; une biomolécule ; ou des combinaisons de ceux-ci.
